# EUROPEAN PATENT APPLICATION

(11) **EP 1 460 476 A1**
(43) Date of publication of application: **22.09.2004**
(21) Application number: 03100663.8
(22) Date of filing: 17.03.2003
(51) Int. Cl.: G03B 42/04, H04N 1/21, A61B 6/00

(54) **Identification method of an image orientation**

(71) Applicant: AGFA-GEVAERT, 2640 Mortsel (BE)
(72) Inventor: Heyns, Guido, c/o Agfa-Gaevert, 2640, Mortsel (BE); Van Riet, Willem, c/o AGfa-Gaevert, 2640 Mortsel (BE)

(57) **Abstract**

In an image identification method a digital signal representation of a marker preferably having an outlook which varies with orientation is coupled to a radiation image so that the quadrant in which the marker is located is invariable.

## Description

### FIELD OF THE INVENTION

The present invention relates to radiation image recording.
More specifically the invention relates to a method of detecting the intrinsic orientation of an object at the time of exposure to radiation.
The method is applicable in medical radiographic image recording applications.

### BACKGROUND OF THE INVENTION

In conventional radiography a radiation image of a patient is obtained by exposing the patient to radiation and by recording the radiation image on a radiographic film.

The film is commonly conveyed in a cassette having in one corner a dedicated identification area that is shielded from radiation during patient exposure.
In an identification station patient identification data are printed on the recording material by exposing the dedicated identification area on the film to the identification data that are provided on a printed identification card.

Depending on the mode of recording (for example anterior-posterior or posterior-anterior) the identification data are printed onto the radiographic film in readable form or in mirrored form.

After film development the radiologist mounts the exposed film on a light box for examination.
Since the radiologists are trained to examine the image in a position as if patient and viewer are facing each other, the film is positioned on the light box so that a face-to-face view is obtained.

For this purpose in some cases, e.g. in the case of an anterior-posterior exposure, the image is to be mirrored before being positioned on the viewing box.

If the image is correctly exposed and correctly identified, the text in the text box will appear in readable form on a pre-defined location relative to the image when it is viewed on a light box.

In case identification and exposure do not match, the radiologist is triggered that an error occurred either at the identification stage or at the exposure stage.

Although the above-described prior art identification system is not full proof (erroneous identification combined with erroneous exposure will not be detectable on the basis of the appearance of the identification data on the film), it can be used as a trigger of mistakes at exposure or identification level.

In digital radiography however the procedure of exposure and identification is different.

A digital signal representation of a radiographic image is generated, for example by the following procedure.
A radiation image of a patient that has been exposed to radiation is temporarily stored in a photo-stimulable phosphor screen. An exposed cassette is then entered into an identification station and patient identification data are written onto the cassette, for example into an EEPROM provided on the cassette.
The exposed screen is then scanned by means of light having a wavelength within the stimulating wavelength range of the phosphor. Light emitted upon stimulation is detected and converted into a signal representation of the radiation image.
The information in the EEPROM is also read out, the read-out data constitute an electronic text tag.

The signal representation can then be processed and applied to a hard copy recorder for generating a hard copy image and/or to a display device for soft copy viewing.

In a digital image acquisition and image processing technique as described higher, the image data and the identification data can be processed independently.
For example in case of an anterior-posterior exposure the image is mirrored - by manipulation of its signal representation - before being applied to a hard copy recorder while the text tag comprising the identification data might not be changed.

As a consequence of the fact that the image data and the identification data can be processed independent from each other, there is no fixed link between the appearance of the image and the appearance of the text tag so that the text tag can no longer be used to trigger the radiologist of occasional mistakes at exposure or identification level.

It is an object of the present invention to overcome the disadvantages of the prior art.

### SUMMARY OF THE INVENTION

The above-mentioned object is realized by a method having the specific features set out in claim 1.

Specific features for preferred embodiments of the invention are set out in the dependent claims.

Further advantages and embodiments of the present invention will become apparent from the following detailed description.

### BRIEF DESCRIPTION OF THE DRAWINGS

Fig. 1 illustrates some examples of images which are provided with a marker,
Fig. 2 shows mirrored images.

### DETAILED DESCRIPTION OF THE INVENTION

According to the present invention a digital representation of a marker preferably having an appearance which varies with orientation is coupled to the digital representation of the radiation image so that the quadrant in which the marker is located is fixed (the same for all images, independent of recording mode or set up).

Additionally the relative orientation of the marker and the image may also be fixed.

Preferably the orientation of the marker depends on the exposure set (e.g. examination type and sub-type) and/or on the orientation of the recording material.

Examples of examination types and sub-types are anterior/posterior or posterior/anterior.
Orientation of the recording material is e.g. landscape or portrait orientation.

The present invention is advantageous in that whenever the marker does not appear on the location corresponding with the envisaged exposure set up and orientation, this triggers the radiologist of the fact that the image may have been manipulated.

It is also advantageous in that whenever the image is zoomed in on the screen or roamed to another position, the marker can move relative to the image so it stays visible in the viewable area of the screen and film while keeping its orientation and/or corner (quadrant where it is positioned) relative to the image.

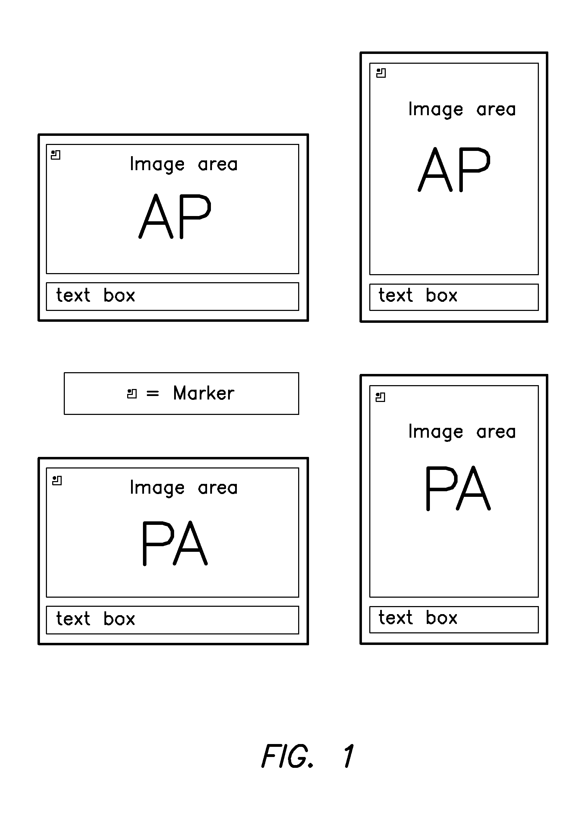

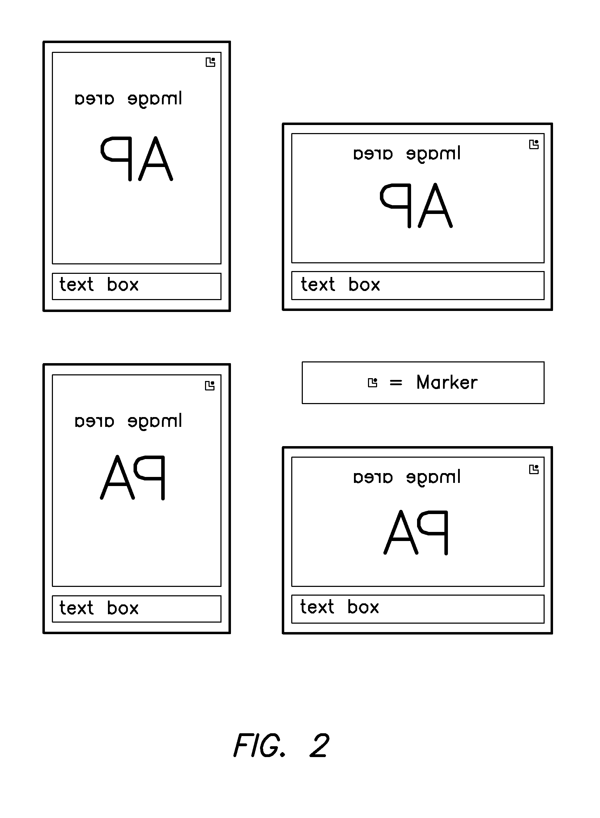

The following examples are illustrated in figures 1 and 2:
* if an image is identified as an anterior - posterior image recorded in landscape mode, the marker is located in the upper left corner of the radiograph.
* if an image is identified as an anterior - posterior image recorded in portrait mode, the marker is also located in the upper left corner of the radiograph.
* if an image is identified as an anterior - posterior landscape image but should have been identified as an anterior - posterior portrait image, the marker will appear in the upper left corner. If in this case the technician would turn the image by processing the digital representation of the image, the marker would be subjected to the same processing since the orientation of the marker is invariable relative to the radiation image. Consequentially the marker will appear on another location than expected so that the radiologist is triggered that the image may have been manipulated through digital image processing.

It is preferable that the outlook of the marker changes when it is turned, flipped, mirrored etc. so that the effect of these operations are visible.

For example an alphanumeric symbol can be used which is e.g. in readable form when the exposure is identified as an anterior - posterior exposure and mirrored when the exposure is identified as a posterior - anterior exposure.

If the image is turned, flipped or put upside - down by manipulation of the digital signal representation of the image, the marker is always subjected to the same type of signal processing as applied to the diagnostic image.

As a consequence the marker may appear on the other side than expected and/or upside down and/or mirrored in case manipulation has occurred.
This will trigger the radiologist.
For example: a rotation of the image of 90 degrees will result in a rotated marker by 90 degrees.

The location of the marker is preferably outside the diagnostic image area and is preferably not covered by any box, ruler or other component.

In a particular embodiment a show/no show option is available permitting the user to prevent display of the marker. However, the possibility to change the data relating to the marker which have been added to the signal representation of the image is not allowable.

In a particular embodiment wherein a radiation image is recorded on a recording member such as a photostimulable phosphor screen conveyed in a cassette the flow of operations is as follows:
- at identification the co-ordinates of the location where the marker is to be positioned are determined in dependence on the identified examination type (AP or PA) and occasionally the cassette orientation (portrait or landscape) so that in a hard or soft copy image the marker will always be located on the same location (e.g. in upper left corner) preferably coupled to the viewable area,
- additionally the orientation of the marker is indicated in dependence on the exposure type (visible or mirrored),
- the image stored in the photostimulable phosphor screen is read out and a digital image representation is generated,
- a composed signal representation is generated representing the image and the marker situated on said location, whereby the mutual orientation of the image and the marker is invariable.

It would also be possible to put a marker on the cassette carrying the photostimulable phosphor screen.

A composed signal as described higher can for example be realized by generating a digital overlay image comprising the marker in a predefined, application and orientation specific position and linking this digital overlay image to the digital image representation of the radiation image so that any orientation manipulations performed on the radiation image are automatically also performed on the overlay image.

As has been described higher, preferably the location of the marker is always the same from the viewing point of the radiologist when he is examining the image on hard or soft copy, e.g. always upper left corner.

Since radiologists are trained to view radiographic images in a face-to-face position (i.e. patient and radiologist facing each other), this means that another corner has to be identified depending on the information whether the image is identified as an AP or PA exposure and as a landscape or a portrait position of the cassette.

The coordinates of the location of the marker will thus preferably depend on the examination set up and on the orientation of the recording member and on the zoom and roaming.

## Claims

1. Method of identifying a radiation image comprising the steps of
- generating a digital signal representation of said radiation image,
- coupling a digital representation of a marker to the digital representation of said radiation image whereby the quadrant in which the marker is located is fixed.

2. A method according to claim 1 wherein the orientation of the marker relative to the radiation image is invariable.

3. A method according to claim 1 or 2 wherein the appearance of said marker changes with rotation.

4. A method according to any of the preceding claims wherein the appearance of said marker changes with mirroring.

5. A method according to any of the preceding claims wherein the orientation of said marker depends on exposure set up and/or orientation of said recording material.

6. A method according to any of the preceding claims wherein in case of image zooming or roaming the marker is moved relative to the image so as to appear in the viewable image area.

7. A method according to claim 1 wherein said radiation image has been recorded on a photostimulable phosphor screen.
